(19) Europäisches Patentamt
European Patent Office
Office européen des brevets

(11) **EP 4 082 441 B1**

(12) **EUROPEAN PATENT SPECIFICATION**

(45) Date of publication and mention
of the grant of the patent:
**06.11.2024 Bulletin 2024/45**

(21) Application number: **20924425.0**

(22) Date of filing: **15.12.2020**

(51) International Patent Classification (IPC):
**A61B 8/08** *(2006.01)* **A61B 8/00** *(2006.01)*
**G16H 30/40** *(2018.01)* **G16H 40/67** *(2018.01)*
**G16H 30/20** *(2018.01)*

(52) Cooperative Patent Classification (CPC):
**A61B 8/485; A61B 8/08; A61B 8/467;**
**A61B 8/5207; A61B 8/5223; G01S 7/52042;**
**G16H 30/20; G16H 30/40; G16H 40/67;**
A61B 8/4405; A61B 8/4472; A61B 8/469;
G16H 50/20

(86) International application number:
**PCT/KR2020/018370**

(87) International publication number:
**WO 2021/182726 (16.09.2021 Gazette 2021/37)**

(54) **ULTRASOUND DIAGNOSTIC APPARATUS AND METHOD FOR OPERATING SAME**

ULTRASCHALLDIAGNOSEVORRICHTUNG UND VERFAHREN ZUM BETRIEB DAVON

APPAREIL DE DIAGNOSTIC ULTRASONORE ET PROCÉDÉ POUR LE FAIRE FONCTIONNER

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.03.2020 KR 20200030755**

(43) Date of publication of application:
**02.11.2022 Bulletin 2022/44**

(73) Proprietor: **Samsung Medison Co., Ltd.**
**Hongcheon-gun, Gangwon-do 25108 (KR)**

(72) Inventors:
• **PARK, Jiyoung**
**Seoul 05340 (KR)**
• **CHOI, Kiwan**
**Seoul 05340 (KR)**

• **OH, Jintaek**
**Seoul 05340 (KR)**

(74) Representative: **Frey, Sven Holger**
**Lorenz & Kollegen**
**Patentanwälte Partnerschaftsgesellschaft mbB**
**Alte Ulmer Strasse 2**
**89522 Heidenheim (DE)**

(56) References cited:
JP-A- 2016 523 167   KR-A- 20140 070 436
KR-A- 20160 117 119   KR-A- 20170 085 516
KR-A- 20170 085 516   KR-B1- 101 649 725
KR-B1- 101 649 725   US-A1- 2016 287 215
US-A1- 2016 331 345   US-A1- 2017 258 438

**Description**

[Technical Field]

Various embodiments relate to an ultrasonic diagnostic apparatus and an operating method thereof, and more specifically, to an ultrasonic diagnostic apparatus capable of accurately and easily acquiring an elasticity value for an object and an operating method thereof.

[Background Art]

In recent years, in the medical field, various medical imaging apparatuses have been widely used for imaging and acquiring information on living tissues of the human body for the purpose of early diagnosis of various diseases or surgery. Representative examples of these medical imaging apparatuses may include an ultrasonic diagnostic apparatus, a computed tomography (CT) apparatus, and a magnetic resonance imaging (MRI) apparatus.

An ultrasonic diagnostic apparatus irradiates an ultrasonic signal generated from a transducer of a probe to an object, and receives information on an echo signal reflected from the object to acquire an image of a portion in the object. Specifically, the ultrasonic diagnostic apparatus is used for medical purposes, such as observation of the inside of the object, detection of foreign substances, injury measurement, and the like. This ultrasonic diagnostic apparatus has advantages in that stability is high, an image can be displayed in real time, and there is safety due to no radiation exposure compared to a diagnostic apparatus using X-rays, and thus is widely used along with other imaging diagnostic apparatuses.

Meanwhile, in order to measure an elasticity value of the object, an elasticity value is acquired at determined measurement positions by acquiring a plurality of elastic images of the object, and respectively determining the measurement positions in the plurality of elastic images according to a user's judgment. In this case, in order to acquire an elasticity value having high reliability, a user should select an appropriate elastic image from among a plurality of acquired elastic images, and select an appropriate measurement position from which the elasticity value is acquired from the selected elastic image, but selecting the elastic image and the measurement position of the elasticity value is not easy. Further, there was a problem in that a process of acquiring the elasticity value while changing the measurement position from which the elasticity value is acquired has to be repeated several times to acquire an elasticity value having high reliability, and thus a lot of time is taken to acquire the elasticity value.

US 2016/331345 A1 provides a method of displaying an elastography image and an ultrasound diagnosis apparatus performing the method. The method includes transmitting an ultrasound signal to an object, receiving a response signal to the transmitted ultrasound signal, determining ultrasound image data based on the received response signal, determining displacement image data based on the determined ultrasound image data, generating elastography images based on the determined displacement image data, dividing the elastography images into sections based on a criterion, and determining a representative elastography image of each of the sections based on one or more elastography images in each of the sections and quality information of the one or more elastography images, the quality information being based on a strain of a first elastography image among the one or more elastography images. The method further includes displaying the representative elastography image.

KR2017 0085516 A relates to an imaging method and apparatus for performing shear wave elastic ultrasound imaging of an observation field in a medium.

[Invention]

[Technical Problem]

Embodiments are directed to providing an ultrasonic diagnostic apparatus capable of providing an elastic image for acquiring a highly reliable elasticity value for an object and information on a measurement position and an operating method thereof. The invention is defined by claim 1. Regarding the operating method of an ultrasound diagnostic apparatus the invention is defined by claim 9.

[Advantageous Effects]

An ultrasonic diagnostic apparatus according to one embodiment can easily and quickly acquire an elasticity value having high reliability for an object by providing an elastic image for acquiring an elasticity value having high reliability and information on a measurement position to a user.

[Description of Drawings]

The present invention may be easily understood by a combination of the following detailed description and the accompanying drawings, in which reference numerals refer to structural elements.

FIG. 1 is a block diagram illustrating a configuration of an ultrasonic diagnostic apparatus according to one embodiment.
FIG. 2 is a set of views illustrating ultrasonic diagnostic apparatuses according to one embodiment.
FIG. 3 is a flow chart illustrating an operating method of the ultrasonic diagnostic apparatus according to one embodiment.
FIGS. 4 and 5 are reference views for describing a method of determining and displaying a recommended elastic image from among a plurality of elastic images by the ultrasonic diagnostic apparatus according to one embodiment.
FIG. 6 is a flow chart illustrating a method of acquiring an elasticity value from a recommended elastic image by the ultrasonic diagnostic apparatus according to one embodiment.
FIG. 7 is a reference view for describing a method of determining a recommended position, from which an elasticity value is acquired, by the ultrasonic diagnostic apparatus according to one embodiment.
FIG. 8 is a view illustrating an example in which the ultrasonic diagnostic apparatus according to one embodiment displays the recommended position for acquiring the elasticity value.
FIG. 9 is a view illustrating an example in which the ultrasonic diagnostic apparatus according to one embodiment displays the recommended position and the elasticity value.
FIG. 10 is a view illustrating an example in which the ultrasonic diagnostic apparatus according to one embodiment displays the elasticity value.
FIG. 11 is a view illustrating an example in which the ultrasonic diagnostic apparatus according to one embodiment displays the recommended position for acquiring the elasticity value.
FIG. 12 is a view illustrating an example in which the ultrasonic diagnostic apparatus according to one embodiment displays the elasticity value.
FIG. 13 is a block diagram illustrating a configuration of an ultrasonic diagnostic apparatus according to one embodiment.

[Best Mode]

An ultrasonic diagnostic apparatus according to one embodiment includes: a display; a memory configured to store one or more instructions; and a processor configured to execute the one or more instructions stored in the memory, wherein the processor acquires a plurality of pieces of elastic data about an object, and then generates a plurality of elastic images based on the plurality of pieces of elastic data, acquires quality information for each of the plurality of elastic images based on the plurality of pieces of elastic data respectively corresponding to the plurality of elastic images, determines at least one recommended elastic image from which the elasticity value is acquired from among the plurality of elastic images based on the quality information, and controls the display to display the at least one determined recommended elastic image.

The processor according to one embodiment acquires reliability information for elasticity values respectively included in the plurality of elastic images, and acquires quality information for each of the plurality of elastic images based on the reliability information.

The processor according to one embodiment determines the quality information of the elastic image based on a proportion of a region where the reliability information is greater than or equal to a preset value from among the entire region of interest from which the elastic data is acquired.

The processor according to one embodiment may determine a recommended position from which the elasticity value is acquired based on reliability information for the elasticity values included in the at least one determined recommended elastic image, and may control the display to display the recommended position on the recommended elastic image.

The ultrasonic diagnostic apparatus according to one embodiment may further include a user input unit configured to receive a user input selecting any one from among the at least one determined recommended elastic image, wherein the processor may determine at least one recommended position from which the elasticity value is acquired based on reliability information for the elasticity values included in the selected recommended elastic image, and may control the display to display the at least one recommended position on the recommended elastic image.

The processor according to one embodiment may control the display to display the priority of the at least one recommended position when the at least one recommended position is a plurality of recommended positions.

The priority of the recommended positions according to one embodiment may be determined based on the quality information corresponding to the elastic image including the recommended position and the reliability information corresponding to the recommended position.

The processor according to one embodiment may acquire the elasticity value corresponding to the at least one recommended position, and may control the display to display the acquired elasticity value.

The processor according to one embodiment may control the display to display the quality information corresponding to the at least one recommended elastic image.

The processor according to one embodiment may determine a representative elasticity value corresponding to the at least one recommended elastic image, and may control the display to display the representative elasticity value.

An operating method of an ultrasonic diagnostic apparatus according to one embodiment includes operations of: acquiring a plurality of pieces of elastic data about an object; generating a plurality of elastic images based on the plurality of pieces of elastic data; acquiring quality information for each of the plurality of elastic images based on the plurality of pieces of elastic data respectively corresponding to the plurality of elastic images; determining at least one recommended elastic image from which an elasticity value is acquired from among the plurality of elastic images based on the quality information; and displaying the at least one determined recommended elastic image.

[Modes of the Invention]

The present specification clarifies the scope of the present invention, as defined by the appended claims, and describes the principle of the present invention and discloses embodiments so that those skilled in the art may implement the present invention. The disclosed embodiments may be implemented in various forms.

The same reference numerals refer to the same components throughout the present specification. The present specification does not describe all elements of the embodiments, and general contents or contents overlapping between the embodiments in the technical field including the present invention will be omitted. The term 'module' or 'unit' used in the specification may be implemented as one or a combination of two or more of software, hardware, and firmware, and a plurality of 'modules' or 'units' may be implemented as one element, or one 'module' or 'unit' may include a plurality of elements according to the embodiments.

Hereinafter, a working principle and embodiments of the present invention will be described with reference to the accompanying drawings.

In the present specification, an image includes a medical image acquired by an ultrasonic imaging apparatus.

In the present specification, an 'object' is an object to be captured, and may include a person, an animal, or a part thereof. For example, the object may include a part of a body (such as a tissue, an organ, or the like), a phantom, or the like.

Throughout the specification, an "ultrasonic image" refers to an image for an object processed based on an ultrasonic signal transmitted to the object and reflected from the object.

Hereinafter, embodiments will be described in detail with reference to the drawings.

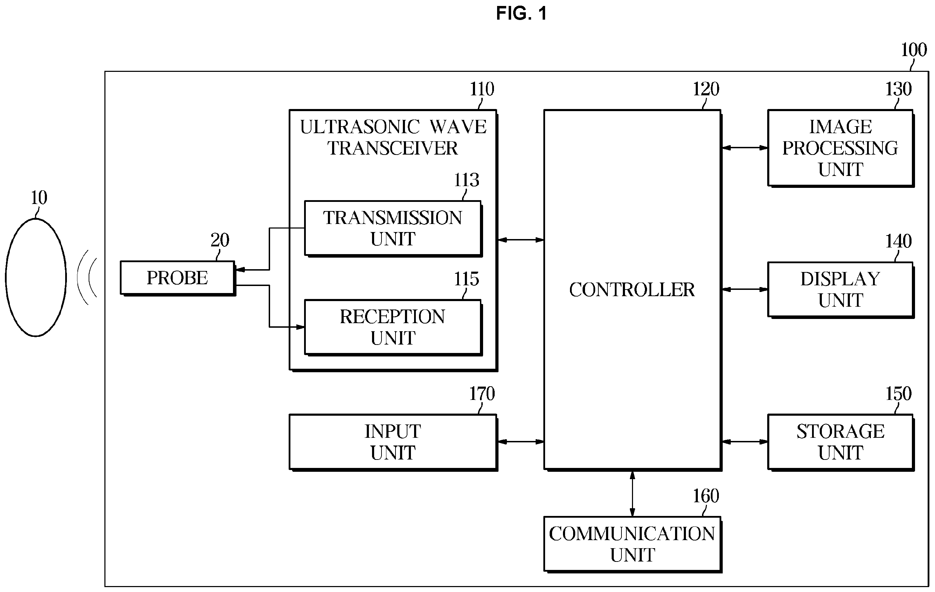

FIG. 1 is a block diagram illustrating a configuration of an ultrasonic diagnostic apparatus 100 according to one embodiment. The ultrasonic diagnostic apparatus 100 according to one embodiment may include a probe 20, an ultrasonic wave transceiver 110, a controller 120, an image processing unit 130, a display unit 140, a storage unit 150, a communication unit 160, and an input unit 170.

The ultrasonic diagnostic apparatus 100 may be implemented not only in a portable type but also a cart type. Examples of the portable ultrasonic diagnostic apparatus may be a smart phone, a laptop computer, a personal digital assistant (PDA), a tablet PC, and the like, which include a probe and an application, but the present invention is not limited thereto.

The probe 20 may include a plurality of transducers. The plurality of transducers may transmit an ultrasonic signal to an object 10 according to a transmission signal applied from a transmission unit 113. The plurality of transducers may receive the ultrasonic signal reflected from the object 10 to form a reception signal. Further, the probe 20 may be integrally implemented with the ultrasonic diagnostic apparatus 100 or may be implemented in a separate type connected to the ultrasonic diagnostic apparatus 100 in a wired or wireless manner. In addition, the ultrasonic diagnostic apparatus 100 may include one probe 20 or a plurality of probes 20 according to an implemented form.

The controller 120 controls the transmission unit 113 to form a transmission signal to be applied to each of the plurality of transducers in consideration of positions and a focal point of the plurality of transducers included in the probe 20.

The controller 120 controls a reception unit 115 to generate ultrasonic data by converting the reception signal received from the probe 20 from an analog signal to a digital signal, and summing the reception signal converted to the digital signal in consideration of the positions and the focal point of the plurality of transducers.

The image processing unit 130 generates the ultrasonic image using the ultrasonic data generated by the ultrasonic wave reception unit 115.

The display unit 140 may display the generated ultrasonic image and various types of information processed by the ultrasonic diagnostic apparatus 100. The ultrasonic diagnostic apparatus 100 may include one or a plurality of display units 140 according to the implemented form. Further, the display unit 140 may be implemented as a touch screen in combination with a touch panel.

The controller 120 may control the overall operation of the ultrasonic diagnostic apparatus 100 and a signal flow between inner components of the ultrasonic diagnostic apparatus 100. The controller 120 may include a memory which stores a program or data for performing a function of the ultrasonic diagnostic apparatus 100, and a processor which processes the program or data. Further, the controller 120 may receive a control signal from the input unit 170 or an external device to control the operation of the ultrasonic diagnostic apparatus 100.

The ultrasonic diagnostic apparatus 100 includes the communication unit 160, and may be connected to an external device (for example, a server, a medical device, a portable device (a smartphone, a tablet PC, a wearable device, or the like)) through the communication unit 160.

The communication unit 160 may include one or more components which enable communication with the external device, and may include, for example, at least one from among a near field communication module, a wired communication module, and a wireless communication module.

The communication unit 160 may transmit and receive the control signal and the data with the external device.

The storage unit 150 may store various types of data or various programs for driving and controlling the ultrasonic diagnostic apparatus 100, ultrasonic data which is input and output, an acquired ultrasonic image, and the like.

The input unit 170 may receive a user's input for controlling the ultrasonic diagnostic apparatus 100. For example, the user's input may include an input which operates a button, a key pad, a mouse, a trackball, a jog switch, a knob, or the like, an input which touches a touch pad or a touch screen, a voice input, a motion input, a biometric information input (for example, iris recognition, fingerprint recognition, or the like), and the like, but the present invention is not limited thereto.

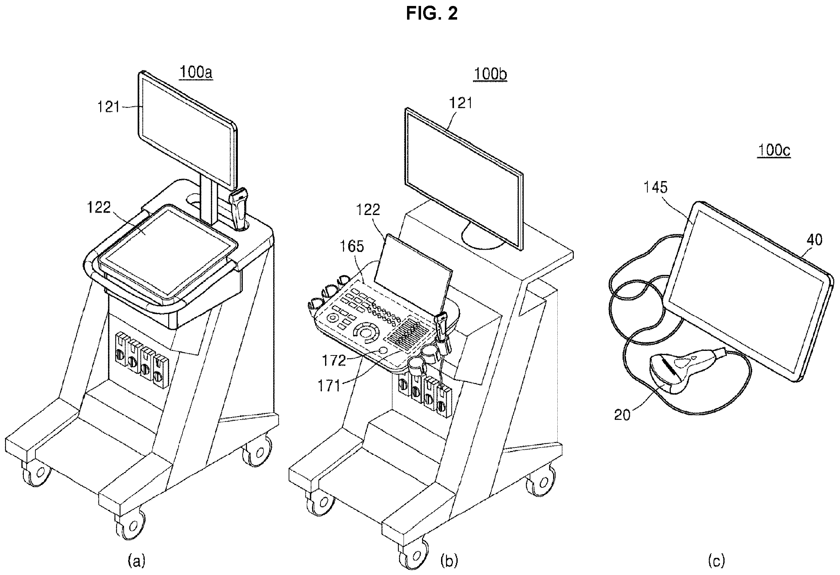

An example of the ultrasonic diagnostic apparatus 100 according to one embodiment will be described below with reference to FIGS. 2A to 2C.

FIGS. 2A to 2C are views illustrating the ultrasonic diagnostic apparatuses according to one embodiment.

Referring to FIGS. 2A and 2B, ultrasonic diagnostic apparatuses 100a and 100b may each include a main display unit 121 and a sub-display unit 122. One of the main display unit 121 and the sub-display unit 122 may be implemented as a touch screen. The main display unit 121 and the sub-display unit 122 may display ultrasonic images or various types of information processed by the ultrasonic diagnostic apparatus 100a or 100b. Further, the main display unit 121 and the sub-display unit 122 are implemented as touch screens, and provide a graphical user interface (GUI) to receive data for controlling the ultrasonic diagnostic apparatus 100a or 100b from a user. For example, the main display unit 121 may display an ultrasonic image, and the sub-display unit 122 may display a control panel for controlling a display of the ultrasonic image in the form of a GUI. The sub-display unit 122 may receive data for controlling the display of an image through the control panel displayed in the form of a GUI. The ultrasonic diagnostic apparatus 100a or 100b may control the display of the ultrasonic image displayed on the main display unit 121 using the received control data.

Referring to FIG. 2B, the ultrasonic diagnostic apparatus 100b may further include a control panel 165 in addition to the main display unit 121 and the sub-display unit 122. The control panel 165 may include a button, a trackball, a jog switch, a knob, and the like, and may receive data for controlling the ultrasonic diagnostic apparatus 100b from a user. For example, the control panel 165 may include a time gain compensation (TGC) button 171, a freeze button 172, and the like. The TGC button 171 is a button for setting a TGC value for each depth of the ultrasonic image. Further, when an input of the freeze button 172 is sensed while scanning an ultrasonic image, the ultrasonic diagnostic apparatus 100b may maintain a state in which a frame image of a corresponding time point is displayed.

Meanwhile, the button, the trackball, the jog switch, the knob, and the like included in the control panel 165 may be provided on the main display unit 121 or the sub-display unit 122 as a GUI.

Referring to FIG. 2C, an ultrasonic diagnostic apparatus 100c may be implemented in a portable type. Examples of the portable ultrasonic diagnostic apparatus 100c may be a smart phone, a laptop computer, a PDA, a tablet PC, and the like, which include a probe and an application, but the present invention is not limited thereto.

The ultrasonic diagnostic apparatus 100c includes a probe 20 and a main body 40, and the probe 20 may be connected to one side of the main body 40 in a wired manner or wireless manner. The main body 40 may include a touch screen 145. The touch screen 145 may display an ultrasonic image, various types of information processed by the ultrasonic diagnostic apparatus, and a GUI.

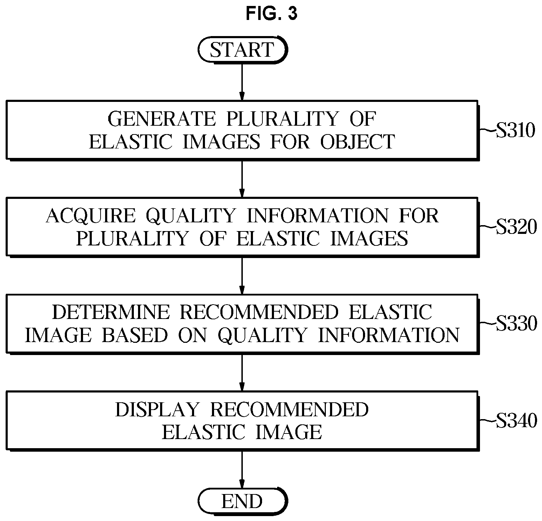

FIG. 3 is a flow chart illustrating an operating method of the ultrasonic diagnostic apparatus according to one embodiment.

The ultrasonic diagnostic apparatus 100 according to one embodiment may acquire ultrasonic data about an object by irradiating an ultrasonic signal to the object and receiving an echo signal for the irradiated ultrasonic signal.

For example, the ultrasonic diagnostic apparatus 100 may acquire B-mode data about the object, and may generate and display a B-mode ultrasonic image based on the B-mode data. The ultrasonic diagnostic apparatus 100 may extract a B-mode component from the ultrasonic data and generate a B-mode image in which signal intensity is expressed as luminance based on the extracted B-mode component.

A region of interest for acquiring elastic data may be set in the B-mode image according to one embodiment, and in this case, a position, a size, and a shape of the region of interest may be variously set. For example, the ultrasonic diagnostic apparatus 100 may receive a user input for setting the region of interest for acquiring the elastic data, and set the region of interest based on the user input.

The ultrasonic diagnostic apparatus 100 according to one embodiment may acquire the elastic data about the region of interest of the object. For example, the ultrasonic diagnostic apparatus 100 may transmit an ultrasonic signal for pushing a partial region (region of interest) of the object to the object, and may cause the displacement of an inner tissue of the object. Since a shear wave is induced in the tissue in the object by the ultrasonic signal, tissue displacement may occur. In one embodiment, the ultrasonic signal for inducing the shear wave may be an acoustic radiation force impulse (ARFI). Since the shear wave is induced in the tissue in the object by the ARFI, shear wave displacement may occur.

The ultrasonic diagnostic apparatus 100 may acquire the elastic data of the object by detecting the shear wave displacement due to the induced shear wave.

The ultrasonic diagnostic apparatus 100 according to one embodiment may generate an elastic image based on the acquired elastic data. In this case, the elastic image may be an image displayed in different colors according to a plurality of elasticity values corresponding to a plurality of points included in the region of interest. For example, a point which is hard and has a low elasticity value, such as a tumor, may be shown in red, and a point corresponding to a relatively soft tissue and having a high elasticity value may be shown in blue.

The ultrasonic diagnostic apparatus 100 according to one embodiment may acquire the elastic data several times to acquire a plurality of elastic images. For example, a first region of interest may be set to acquire first elastic data corresponding to the first region of interest, and a second first region of interest different from the first region of interest may be set to acquire second elastic data. In this case, the first region of interest and the second region of interest may be different from each other in terms of at least one among a position, a size, and a shape. Like the above, the ultrasonic diagnostic apparatus 100 may acquire a plurality of pieces of elastic data corresponding to a plurality of regions of interest while setting the regions of interest differently, and may generate the plurality of elastic images based on the plurality of pieces of elastic data (S310).

The ultrasonic diagnostic apparatus 100 according to one embodiment acquires quality information for the plurality of elastic images (S320).

In this case, the quality information of the elastic images may be information which indicates a reliability index (the reliability of the elastic images) of the elastic images. For example, the elastic image includes less noise information as the quality information of the elastic image is higher, and it may be determined as an appropriate elastic image for measuring an elasticity value. On the other hand, the elastic image includes more noise information as the quality information of the elastic image is lower, and it may be determined as an inappropriate elastic image for measuring the elasticity value.

For example, the ultrasonic diagnostic apparatus 100 may calculate quality information of the elastic images based on reliability information for the elasticity values included in the elastic images. In this case, the elasticity value may refer to a value which indicates a degree of elasticity of the tissue of the object, and the reliability information for the elasticity value may be a value which indicates the reliability of the elasticity value calculated by the ultrasonic diagnostic apparatus. The reliability information may include a reliable measurement index or a measurement reliability index, but the present invention is not limited thereto.

The ultrasonic diagnostic apparatus 100 determines the quality information of the elastic images based on a proportion of a region where the reliability information is greater than or equal to a threshold value from among the entire region of interest from which the elastic data is acquired. For example, the quality information of the elastic images may be defined as in the following Equation 1.

[Equation 1]

$$\mathbf{quality\ index = weight} \times \frac{\mathbf{B}}{\mathbf{A}}$$

In Equation 1, quality index indicates the quality information of the elastic images, A indicates an area of the entire region of interest from which the elastic data is acquired, and B indicates an area of a region where the reliability information is greater than or equal to a threshold value from among the region of interest. In this case, the threshold value may be a value set by a user input or may be a preset value. Further, weight indicates a weight, and the weight may be determined by various parameters. For example, the weight may be determined in proportion to the magnitude of the shear wave induced in the object. Alternatively, the weight may be determined to be large as the magnitude of a measurement signal for observing the shear wave is large and a value of a standard deviation is small. Alternatively, the weight may be determined to be small as a standard deviation of the elasticity values included in the region of interest is large. Alternatively, the weight may be determined to be large based on an average value of the elasticity values included in the region of interest or average reliability information. Alternatively, when the elastic data is acquired, the weight may be determined according to a contact state of the probe. However, the present invention is not limited thereto.

The ultrasonic diagnostic apparatus 100 according to one embodiment may display the quality information acquired from the plurality of elastic images. For example, the plurality of elastic images may be displayed, and the quality information may be displayed as a numerical value on each of the plurality of elastic images. In this case, the numerical value may be a value calculated according to Equation 1, but is not limited thereto. Further, the quality information may be displayed in various ways such as a color, a graph, or the like. For example, the color displayed on the elastic image may be different according to a value which indicates the quality information.

The ultrasonic diagnostic apparatus 100 according to one embodiment determines at least one recommended elastic image based on the quality information for the plurality of elastic images (S330).

The ultrasonic diagnostic apparatus 100 determines at least one elastic image having quality information greater than or equal to a threshold value from among the plurality of elastic images as the recommended elastic image. In this case, the threshold value for the quality information is a value set by a user input.

The ultrasonic diagnostic apparatus 100 according to one embodiment displays the at least one recommended elastic image on a display (S340).

In this case, when the recommended elastic image is a plurality of recommended elastic images, the ultrasonic diagnostic apparatus may display the recommended elastic images in the display in the order of an increasing value representing quality information. Further, the ultrasonic diagnostic apparatus 100 may also display the quality information of the recommended elastic image.

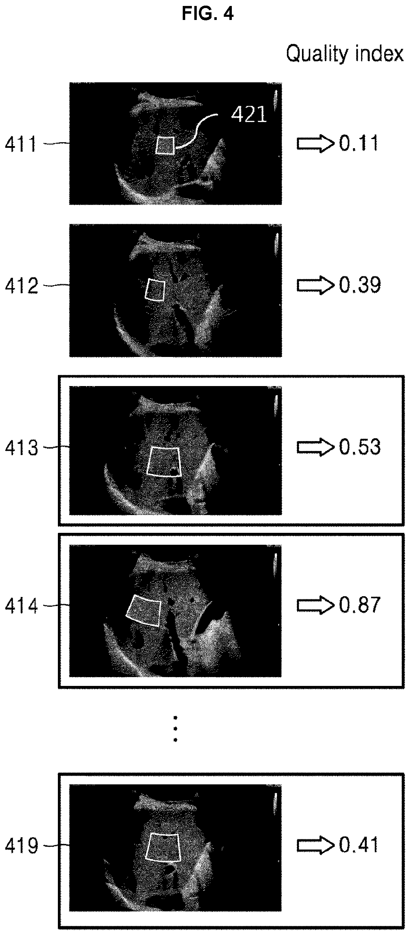

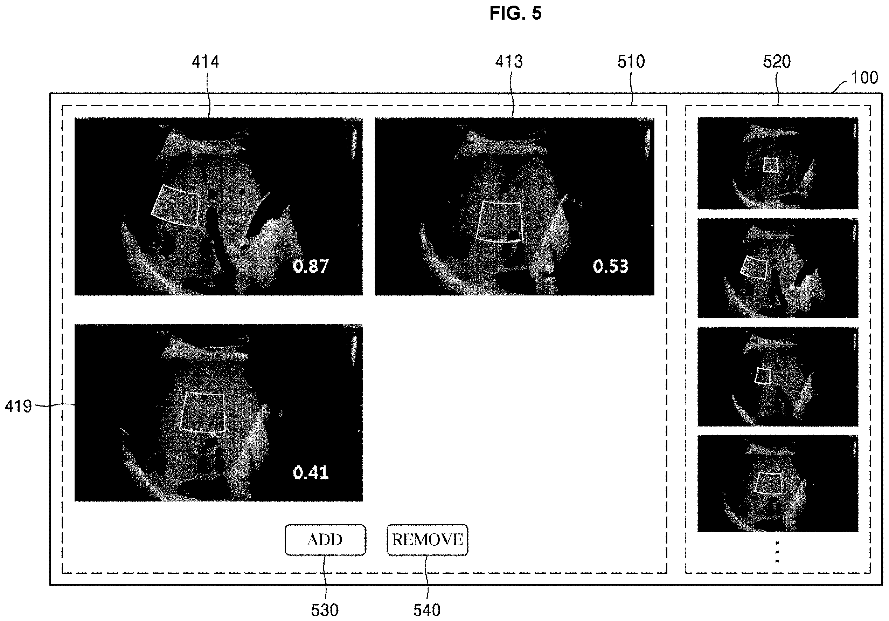

FIGS. 4 and 5 are reference views for describing a method of determining and displaying a recommended elastic image from among a plurality of elastic images by the ultrasonic diagnostic apparatus according to one embodiment.

Referring to FIG. 4, the ultrasonic diagnostic apparatus 100 according to one embodiment may generate a plurality of elastic images 411, 412, 413, 414, ..., and 419. For example, the ultrasonic diagnostic apparatus 100 may acquire a plurality of pieces of elastic data and generate the plurality of elastic images 411, 412, 413, 414, ..., and 419 based on the plurality of pieces of elastic data. Since the above is described in detail in FIG. 3, the same description will be omitted.

The ultrasonic diagnostic apparatus 100 may determine quality information for each of the plurality of elastic images 411, 412, 413, 414, ..., and 419. For example, the ultrasonic diagnostic apparatus 100 may calculate reliability information for elasticity values included in a first elastic image 411. The ultrasonic diagnostic apparatus 100 may calculate the quality information of the first elastic image 411 (for example, a quality index is 0.11) based on a proportion of a region where the reliability information is greater than or equal to a threshold value (for example, 0.9) from among the entire region of interest 421 from which elastic data is acquired.

The ultrasonic diagnostic apparatus 100 may calculate the quality information for the remaining elastic images 412, 413, 414, ..., and 419 in the same manner. The ultrasonic diagnostic apparatus 100 may determine elastic images for which the quality information values are greater than or equal to a threshold value (for example, 0.4) from among the plurality of elastic images 411, 412, 413, 414, ..., and 419 as recommended elastic images.

For example, as shown in FIG. 4, a third elastic image 413, a fourth elastic image 414, and a fifth elastic image 419 may be determined as recommended elastic images.

Referring to FIG. 5, the ultrasonic diagnostic apparatus 100 according to one embodiment may display the recommended elastic images on a display. For example, the ultrasonic diagnostic apparatus 100 may display the third elastic image 413, the fourth elastic image 414, and the fifth elastic image 419 on a first region 510 of the display. Further, the plurality of elastic images 411, 412, 413, 414, ..., and 419 may be displayed on a second region 520 of the display in a list form. However, the present invention is not limited thereto.

The ultrasonic diagnostic apparatus 100 may display the third elastic image 413, the fourth elastic image 414, and the fifth elastic image 419 determined as the recommended elastic images in the order of an increasing quality information value. For example, the ultrasonic diagnostic apparatus 100 may display in the order of the fourth elastic image 414, the third elastic image 413, and the fifth elastic image 419. In this case, the ultrasonic diagnostic apparatus 100 may also display the quality information corresponding to the recommended elastic images.

Further, the ultrasonic diagnostic apparatus 100 may provide a user interface for adding or removing a recommended elastic image. For example, the ultrasonic diagnostic apparatus 100 may display an adding item 530 and a removing item 540 as shown in FIG. 5, and may display an elastic image having the largest quality information value from among the plurality of elastic images not determined as the recommended elastic images in the first region 510 when the adding item 530 is selected. For example, when the adding item 530 is selected in a state in which the third to fifth elastic images 413, 414, and 419 are displayed, the ultrasonic diagnostic apparatus 100 may display a second elastic image 412 having the largest quality information value after the fifth elastic image from among the plurality of elastic images in the first region 510.

Further, the ultrasonic diagnostic apparatus 100 may not display an elastic image having the smallest quality information value from among the recommended elastic images displayed in the first region 510 when the removing item 540 is selected. For example, when the removing item 540 is selected in the state in which the third to fifth elastic images 413, 414, and 419 are displayed, the ultrasonic diagnostic apparatus 100 may control the display so that the fifth elastic image 419 is not displayed in the first region 510.

Alternatively, although not shown in the drawings, the ultrasonic diagnostic apparatus 100 may determine the number of recommended elastic images by setting a threshold value for the reliability information or a threshold value for the quality information of the elastic images based on a user input which sets the threshold value for the reliability information or the threshold value for the quality information of the elastic images. Alternatively, the ultrasonic diagnostic apparatus 100 may determine the number of recommended elastic images based on the user input which sets the number of recommended elastic images. However, the present invention is not limited thereto.

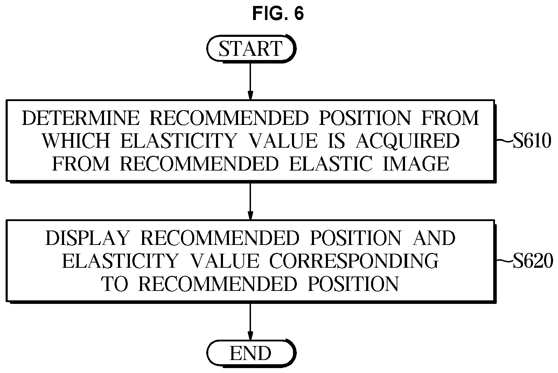

FIG. 6 is a flow chart illustrating a method of acquiring an elasticity value from a recommended elastic image by the ultrasonic diagnostic apparatus according to one embodiment.

Referring to FIG. 6, the ultrasonic diagnostic apparatus 100 according to one embodiment may determine a recommended position from which the elasticity value is acquired from the recommended elastic image (S610).

For example, the ultrasonic diagnostic apparatus 100 may determine a plurality of regions of interest for calculating the elasticity value and the reliability information for the elasticity value in the recommended elastic image. The ultrasonic diagnostic apparatus 100 may calculate the elasticity value for each of the regions of interest based on the acquired elastic data, and may calculate the reliability information for the calculated elasticity value.

When the reliability information is greater than or equal to a threshold value, the ultrasonic diagnostic apparatus 100 may determine the region of interest as the recommended position.

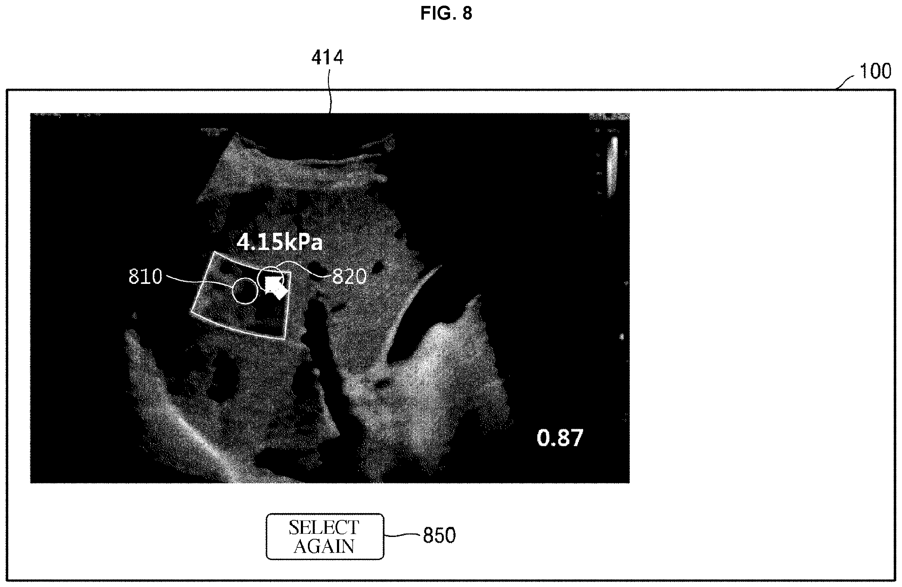

When the recommended position is determined, the ultrasonic diagnostic apparatus 100 according to one embodiment may display the recommended position on the recommended elastic image and display the elasticity value corresponding to the recommended position (S620). The above will be described in detail with reference to FIGS. 8 to 12.

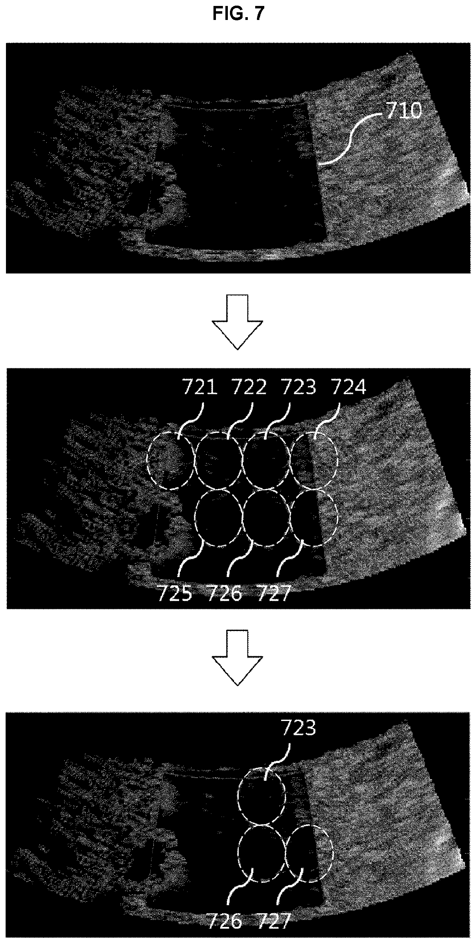

FIG. 7 is a reference view for describing a method of determining the recommended position, from which the elasticity value is acquired, by the ultrasonic diagnostic apparatus according to one embodiment.

Referring to FIG. 7, the ultrasonic diagnostic apparatus 100 according to one embodiment may divide a region of interest from which the elastic data is acquired into a plurality of sub-regions of interest 721, 722, 723, 724, 725, 726, and 727. In this case, the number, sizes, shapes, and the like of the plurality of sub-regions of interest 721, 722, 723, 724, 725, 726, and 727 may be variously determined.

The ultrasonic diagnostic apparatus 100 according to one embodiment may calculate elasticity values respectively corresponding to the plurality of sub-regions of interest 721, 722, 723, 724, 725, 726, and 727, and reliability information for the elasticity values based on the acquired elastic data. In this case, the elasticity value refers to a value which indicates a degree of elasticity of a tissue corresponding to the sub-region of interest, and the reliability information may refer to a value which indicates the reliability of the calculated elasticity value. The reliability information may be a value which indicates a degree to which a shear wave satisfies a wave equation when the shear wave induced by the ultrasonic data to acquire the elastic data satisfies the wave equation. However, the present invention is not limited thereto, and the reliability information may be calculated in various ways.

The ultrasonic diagnostic apparatus 100 may determine the recommended positions based on the reliability information for the elasticity values corresponding to the plurality of sub-regions of interest 721, 722, 723, 724, 725, 726, and 727. For example, the ultrasonic diagnostic apparatus 100 may determine the sub-regions having reliability information greater than or equal to a threshold value as a recommended position from which the elasticity value is acquired, and as shown in FIG. 7, a third sub-region of interest 723, a sixth sub-region of interest 726, and a seventh sub-region of interest 727 having the reliability information greater than or equal to 0.9 may be determined as the recommended positions.

Alternatively, the ultrasonic diagnostic apparatus 100 may determine, a preset number of sub-regions of interest in the order of increasing reliability information from among the plurality of sub-regions of interest as the recommended positions.

FIG. 8 is a view illustrating an example in which the ultrasonic diagnostic apparatus according to one embodiment displays the recommended position for acquiring the elasticity value.

As shown in FIG. 8, when the recommended elastic images are displayed, and then an input selecting any one of the recommended elastic images is received, the ultrasonic diagnostic apparatus 100 according to one embodiment may display the recommended position on the selected elastic image.

For example, among first to third recommended elastic images 414, 413, and 419, when an input selecting the first recommended elastic image 414 is received, recommended positions 810 and 820 for acquiring the elasticity values may be displayed on the first recommended elastic image 414 while enlarging and displaying the first recommended elastic image 414. Since the method of determining the recommended position in the elastic image is described in FIG.

7, the detailed description thereof will be omitted.

The ultrasonic diagnostic apparatus 100 according to one embodiment may guide a user to the measurement position where an elasticity value having high reliability may be acquired by displaying the recommended position.

When an input selecting any one of the displayed recommended positions 810 and 820 is received, the ultrasonic diagnostic apparatus 100 may display an elasticity value corresponding to the selected recommended position. For example, as shown in FIG. 8, when an input selecting the second recommended position 820 is received, the ultrasonic diagnostic apparatus 100 may display a second elasticity value (for example, 4.15 kPa) corresponding to the second recommended position 820. Further, although not shown in the drawings, the ultrasonic diagnostic apparatus 100 may also display reliability information for the second elasticity value.

In addition, the ultrasonic diagnostic apparatus 100 according to one embodiment may display an item 850 for selecting the elastic image again, and when the item 850 is selected, as shown in FIG. 8, the recommended elastic image may be displayed again.

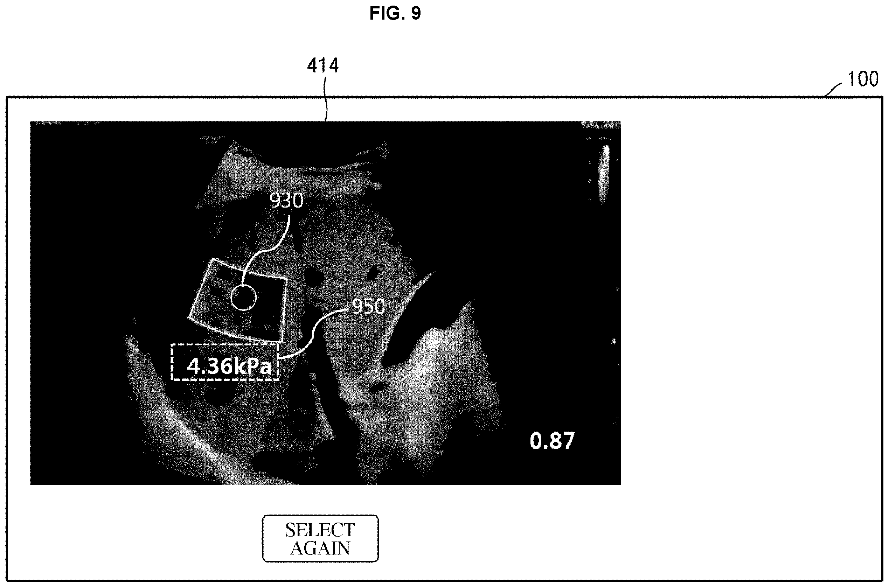

FIG. 9 is a view illustrating an example in which the ultrasonic diagnostic apparatus according to one embodiment displays the recommended position and the elasticity value.

As shown in FIG. 8, when the recommended elastic images are displayed, and then an input selecting any one of the recommended elastic images is received, the ultrasonic diagnostic apparatus 100 according to one embodiment may display the recommended position and the elasticity value corresponding to the recommended position on the selected elastic image.

For example, when an input selecting the first recommended elastic image 414 from among the first to third recommended elastic images 414, 413, and 419 is received, a sub-region of interest having the largest reliability information may be displayed as a recommended position 930 in the first recommended elastic image 414 while enlarging and displaying the first recommended elastic image 414. Further, an elasticity value 950 corresponding to the recommended position may also be displayed.

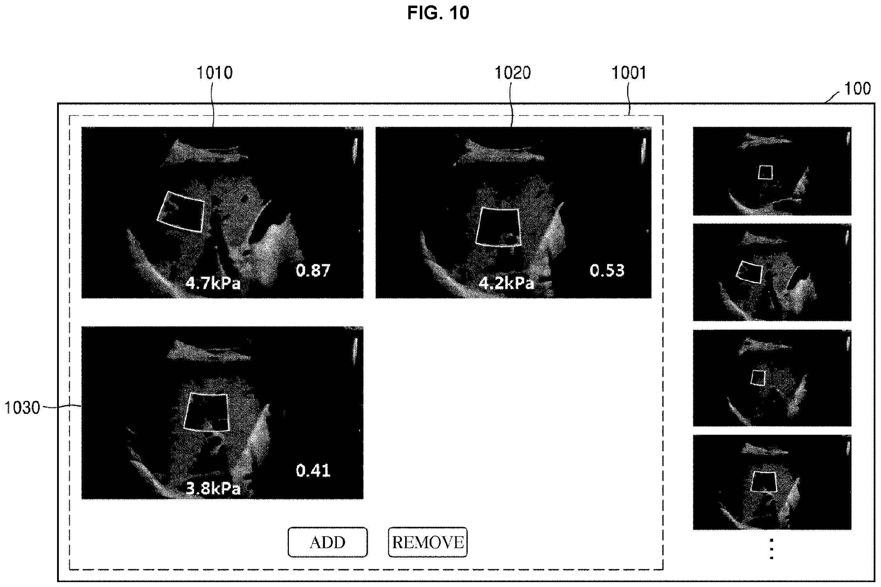

FIG. 10 is a view illustrating an example in which the ultrasonic diagnostic apparatus according to one embodiment displays the elasticity value.

Referring to FIG. 10, the ultrasonic diagnostic apparatus 100 according to one embodiment may display recommended elastic images 1010, 1020, and 1030 on a display. For example, the ultrasonic diagnostic apparatus 100 may display the elastic images 1010, 1020, and 1030 determined as recommended elastic images from among the plurality of elastic images in a first region 1001 of the display. Since the method of determining the recommended elastic images from among the plurality of elastic images and displaying the determined elastic images is described in detail in FIGS. 4 and 5, the detailed description thereof will be omitted.

The ultrasonic diagnostic apparatus 100 may acquire representative elasticity values respectively corresponding to the recommended elastic images 1010, 1020, and 1030. For example, the ultrasonic diagnostic apparatus 100 may determine the representative elasticity values based on the elasticity values included in the recommended elastic images and the reliability information for the elasticity values. The ultrasonic diagnostic apparatus 100 may determine an average value of the elasticity values having the reliability information greater than or equal to a threshold value from among the elasticity values included in the recommended elastic image as a representative elasticity value corresponding to the recommended elastic image. However, the present invention is not limited thereto, and the representative elasticity values may be acquired in various ways.

The ultrasonic diagnostic apparatus 100 may display the representative elasticity values corresponding to the recommended elastic images. For example, a first representative elasticity value (for example, 4.7 kPa) corresponding to a first recommended elastic image 1010, a second representative elasticity value (for example, 4.2 kPa) corresponding to a second recommended elastic image 1020, and a third representative elasticity value (for example, 3.8 kPa) corresponding to a third recommended elastic image 1030 may be displayed.

Since the ultrasonic diagnostic apparatus 100 simultaneously provides the recommended elastic images and the representative elasticity values, a user may easily understand the representative elasticity values for the recommended elastic images at once without selecting a specific elastic image and selecting an elasticity value measurement position.

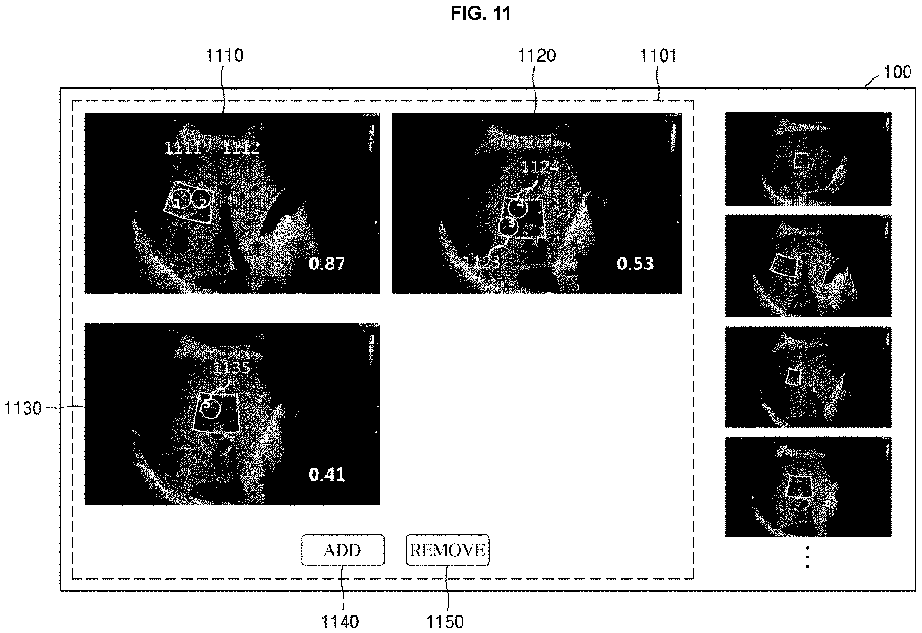

FIG. 11 is a view illustrating an example in which the ultrasonic diagnostic apparatus according to one embodiment displays the recommended position for acquiring the elasticity value.

Referring to FIG. 11, the ultrasonic diagnostic apparatus 100 according to one embodiment may display recommended elastic images on a display. For example, the ultrasonic diagnostic apparatus 100 may display elastic images 1110, 1120, and 1130 determined as recommended elastic images from among the plurality of elastic images in a first region 1101 of the display. Since the method of determining the recommended elastic images from among the plurality of elastic images and displaying the determined elastic images is described in detail in FIGS. 4 and 5, the detailed description thereof will be omitted.

The ultrasonic diagnostic apparatus 100 may determine at least one recommended position from which the elasticity value is acquired from each of the recommended elastic images. Since the method of determining the at least one recommended position from which the elasticity value is acquired is described in detail in FIG. 7, the detailed description thereof will be omitted. The ultrasonic diagnostic apparatus 100 may display the determined recommended position on the recommended elastic image. For example, a first recommended position 1111 and a second recommended position 1112 may be displayed on a first recommended elastic image 1110, a third recommended position 1123 and a fourth recommended position 1124 may be displayed on a second recommended elastic image 1120, and a fifth recommended position 1135 may be displayed on a third recommended elastic image 1130.

In this case, the ultrasonic diagnostic apparatus 100 may determine the priority of the plurality of recommended positions based on the quality information of the elastic images including the recommended positions and the reliability information corresponding to the recommended positions. Further, as shown in FIG. 11, the ultrasonic diagnostic apparatus 100 may display the priority of the recommended positions 1111, 1112, 1123, 1124, and 1135. For example, a number corresponding to the priority may be displayed in the recommended position, and the priority is higher as the number is smaller. The quality information of the first recommended elastic image 1110 including the first recommended position 1111 at which the number '1' is displayed may be the best, and the reliability information for the elasticity value corresponding to the first recommended position 1111 may be the largest.

Further, the ultrasonic diagnostic apparatus 100 may provide a user interface for adding or removing a recommended position. For example, the ultrasonic diagnostic apparatus 100 may display an adding item 1140 and a removing item 1150 as shown in FIG. 11, and may display a sub-region of interest having the largest reliability information from among a plurality of sub-regions of interest not determined as the recommended position when the adding item 1140 is selected.

For example, when the adding item 1140 is selected in a state in which first to fifth recommended positions 1111, 1112, 1123, 1124, and 1135 are displayed, the sub-region of interest having the largest reliability information from among the plurality of sub-regions of interest not determined as the recommended position may be determined and displayed as a sixth recommended position. In this case, when the sixth recommended position is included in the previously displayed first to third recommended elastic images 1110, 1120, and 1130, the sixth recommended position may be displayed on the corresponding recommended elastic image, and when the sixth recommended position is included in the fourth elastic image not previously displayed as the recommended elastic image, the fourth elastic image may be displayed in the first region 1101, and the sixth recommended position may be displayed on the fourth elastic image.

Further, when the removing item 1150 is selected, the ultrasonic diagnostic apparatus 100 may not display a recommended position having the lowest priority from among the displayed recommended positions. For example, when the removing item 1150 is selected in the state in which the first to fifth recommended positions 1111, 1112, 1123, 1124, and 1135 are displayed, the ultrasonic diagnostic apparatus 100 may control the display so that so that the fifth recommended position 1135 having the lowest priority is not displayed. In addition, the display may be controlled so that the third recommended elastic image 1130 including only the fifth recommended position 1135 is not displayed.

Alternatively, although not shown in the drawings, the ultrasonic diagnostic apparatus 100 may adjust the number of recommended positions based on a user input which sets the threshold value for the reliability information for determining the recommended positions. Alternatively, the number of recommended positions may be determined based on a user input which sets the number of recommended positions. However, the present invention is not limited thereto.

When an input selecting any one of the displayed recommended positions is received, the ultrasonic diagnostic apparatus 100 may acquire and display the elasticity value corresponding to the selected recommended position. Further, the ultrasonic diagnostic apparatus 100 may also display the reliability information for the elasticity value corresponding to the recommended position.

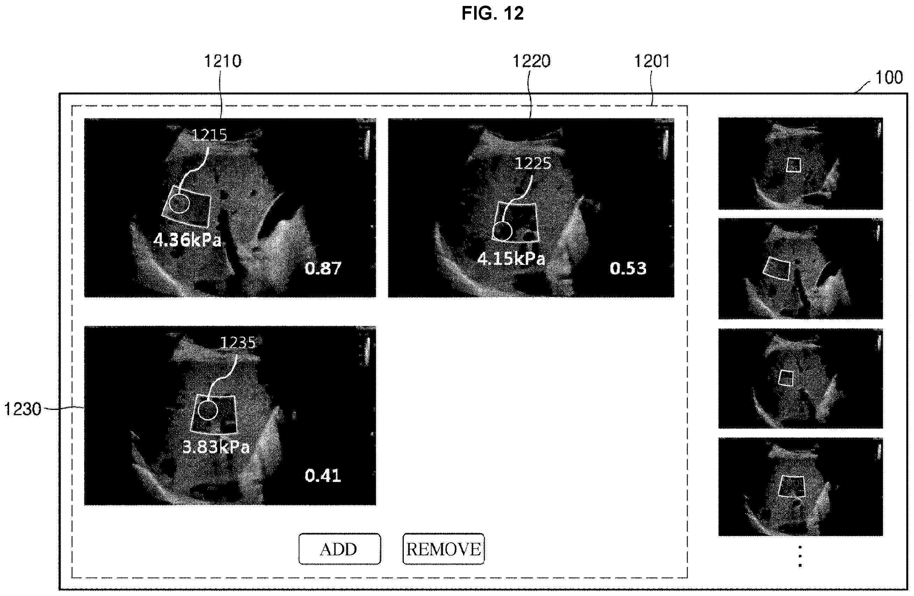

FIG. 12 is a view illustrating an example in which the ultrasonic diagnostic apparatus according to one embodiment displays the elasticity value.

Referring to FIG. 12, the ultrasonic diagnostic apparatus 100 according to one embodiment may display recommended elastic images on a display. For example, the ultrasonic diagnostic apparatus 100 may display elastic images 1210, 1220, and 1230 determined as recommended elastic images from among the plurality of elastic images in a first region 1201 of the display. Since the method of determining the recommended elastic images from among the plurality of elastic images and displaying the determined elastic images is described in detail in FIGS. 4 and 5, the detailed description thereof will be omitted.

The ultrasonic diagnostic apparatus 100 may determine representative measurement positions respectively corresponding to the recommended elastic images 1210, 1220, and 1230. For example, the ultrasonic diagnostic apparatus 100 may determine a sub-region of interest having the highest reliability information from among the sub-regions of interest included in the recommended elastic image as the representative measurement position.

The ultrasonic diagnostic apparatus 100 may display the representative measurement positions on each of the recommended elastic images, and display an elasticity value corresponding to the representative measurement position. For example, the ultrasonic diagnostic apparatus 100 may display an elasticity value (for example, 4.36 kPa) acquired at a first representative measurement position 1215 in addition to the first representative measurement position 1215 on a first recommended elastic image 1210, may display an elasticity value (for example, 4.15 kPa) acquired at a second representative measurement position 1225 in addition to the second representative measurement position 1225 on a second recommended elastic image 1220, and may display an elasticity value (for example, 3.83 kPa) acquired at a third representative measurement position 1235 in addition to the third representative measurement position 1235 on a third recommended elastic image 1230.

Since the ultrasonic diagnostic apparatus 100 simultaneously provides the recommended elastic images, the representative measurement position for each of the recommended elastic images, and the elasticity value corresponding to the representative measurement position, the user may easily understand the representative elasticity values for the recommended elastic images at once without selecting a specific elastic image and selecting an elasticity value measurement position.

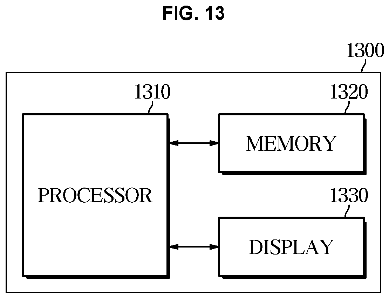

FIG. 13 is a block diagram illustrating a configuration of an ultrasonic diagnostic apparatus according to one embodiment.

Referring to FIG. 13, an ultrasonic diagnostic apparatus 1300 according to one embodiment may include a processor 1310, a memory 1320, and a display 1330.

The processor 1310 in FIG. 13 may correspond to at least one of the ultrasonic wave transceiver 110, the controller 120, and the image processing unit 130 in FIG. 1 or a combination thereof, and the display 1330 may correspond to the display unit 140 in FIG. 1. Further, according to the embodiment, some of the components of the ultrasonic diagnostic apparatus 100 shown in FIG. 1 may be included in the ultrasonic diagnostic apparatus 1300 shown in FIG. 13.

The processor 1310 according to one embodiment may generally control the ultrasonic diagnostic apparatus 1300. The processor 1310 according to one embodiment may execute one or more programs stored in the memory 1320.

The memory 1320 according to one embodiment may store various types of data, various programs, or various applications for driving and controlling the ultrasonic diagnostic apparatus 1300. A program stored in the memory 1320 may include one or more instructions. The program (one or more instructions) or application stored in the memory 1320 may be executed by the processor 1310.

The processor 1310 according to one embodiment may transmit an ultrasonic signal to an object, may acquire ultrasonic data based on an echo signal received from the object, and may generate a B-mode ultrasonic image of the object based on the ultrasonic data. Further, when a region of interest for acquiring elastic data is set based on the B-mode ultrasonic image, the processor 1310 may acquire the elastic data about the region of interest. Since the processor 1310 transmits the ultrasonic signal to the object, and a shear wave may be induced in a tissue in the object by the ultrasonic signal, displacement of the tissue may occur. The processor 1310 may acquire the elastic data of the object by detecting shear wave displacement due to the induced shear wave, and may generate the elastic image based on the acquired elastic data.

The processor 1310 according to one embodiment may acquire the elastic data several times to acquire a plurality of elastic images.

The processor 1310 may acquire quality information for the plurality of elastic images. In this case, the quality information of the elastic images may be information which indicates a reliability index (the reliability of the elastic images) of the elastic images. For example, the elastic image includes less noise information as the quality information of the elastic image is higher, and may be determined as an appropriate elastic image for measuring an elasticity value. On the other hand, the elastic image includes more noise information as the quality information of the elastic image is lower, and may be determined as an inappropriate elastic image for measuring the elasticity value. For example, the processor 1310 may calculate the quality information of the elastic images based on reliability information on elasticity values included in the elastic images.

The processor 1310 determines at least one recommended elastic image based on the quality information for the plurality of elastic images. The processor 1310 determines at least one elastic image having quality information greater than or equal to a threshold value from among the plurality of elastic images as the recommended elastic image. In this case, the threshold value for the quality information is a value set by a user input.

The processor 1310 may control the display 1330 to display the at least one recommended elastic image.

The display 1330 according to one embodiment may display an operation state of the ultrasonic diagnostic apparatus 1300, an ultrasonic image, a user interface, and the like. The display 1330 may include one or more display panels according to the embodiment, and the display 1330 may be implemented in the form of a touch screen.

The display 1330 according to one embodiment may display at least one recommended elastic image from among the plurality of acquired elastic images. Further, when the recommended elastic image is a plurality of recommended elastic images, the display 1330 may display the recommended elastic images on the display in the order of an increasing value representing quality information, and may also display the quality information of the recommended elastic images. Since the above is described in detail in FIG. 5, the detailed description thereof will be omitted.

Further, the processor 1310 according to one embodiment may determine a recommended position from which the elasticity value is acquired from the recommended elastic image. For example, the processor 1310 may divide a region of interest from which the elastic data is acquired into a plurality of sub-regions of interest, and may calculate elasticity values corresponding to the plurality of sub-regions of interest and reliability information. The processor 1310 may determine the recommended position from which the elasticity value is acquired based on the elasticity values corresponding to the plurality of sub-regions of interest and the reliability information.

Further, when the recommended position is determined, the display 1330 according to one embodiment may display the recommended position on the recommended elastic image. In addition, the display 1330 according to one embodiment may display the elasticity value corresponding to the recommended position. Since the above is described in detail in FIGS. 8 to 12, the detailed description thereof will be omitted.

Meanwhile, the block diagrams of the ultrasonic diagnostic apparatuses 100 and 1300 shown in FIGS. 1 and 13 are block diagrams for one embodiment. Each component in the block diagrams may be integrated, added, or omitted according to the specifications of the ultrasonic diagnostic apparatuses 100 and 1300 which are actually implemented. That is, two or more components may be combined into one component, or one component may be divided into two or more components as necessary. Further, a function performed in each block is provided to describe the embodiments, and the specific operation or apparatus does not limit the scope of the present invention.

The operating method of the ultrasonic diagnostic apparatus according to one embodiment may be implemented in the form of program instructions which may be executed through various computer means and recorded in a computer-readable medium. The computer-readable medium may include program instructions, data files, data structures, and the like alone or in a combination thereof. The program instructions recorded in the medium may be specially designed and configured for the present invention, or may be known to those skilled in the art of computer software and usable. Examples of a computer-readable recording medium include magnetic media such as a hard disk, a floppy disk, and a magnetic tape, optical media such as a CD-read-only memory (ROM) and a digital versatile disc (DVD), and magneto-optical media such as a floptical disk, and hardware devices specially configured to store and execute the program instructions such as a ROM, a random access memory (RAM), a flash memory, and the like. Examples of the program instructions include not only machine language code such as code generated by a compiler, but also high-level language code which may be executed by a computer using an interpreter or the like.

Further, the ultrasonic diagnostic apparatus according to the disclosed embodiments and the operating method of the ultrasonic diagnostic apparatus may be included in a computer program product and provided. The computer program product may be traded between a seller and a buyers as a commodity.

The computer program product may include a software (S/W) program and a computer-readable storage medium in which the S/W program is stored. For example, the computer program product may include a product (for example, downloadable apps) in a form of the S/W program electronically distributed through a manufacturer of electronic devices or electronic markets (for example, Google Play Store, App Store). For electronic distribution, at least a portion of the S/W program may be stored in the storage medium or may be temporarily generated. In this case, the storage medium may be a storage medium of a server of a manufacturer, a server of an electronic market, or a relay server which temporarily stores the SW program.

The computer program product, in a system composed of a server and a client device, may include a storage medium of the server or a storage medium of the client device. Alternatively, when there is a third device (for example, a smart phone) communicatively connected to the server or the client device, the computer program product may include a storage medium of the third device. Alternatively, the computer program product may include the S/W program itself transmitted from the server to the client device or the third device, or transmitted from the third device to the client device.

In this case, one of the server, the client device, and the third device may execute the computer program product to perform the methods according to the disclosed embodiments. Alternatively, two or more of the server, the client device, and the third device may execute the computer program product to distribute and execute the methods according to the disclosed embodiments.

For example, a server (for example, a cloud server, an artificial intelligence server, or the like) may execute the computer program product stored in the server to control the client device communicatively connected with the server to perform the methods according to the disclosed embodiments.

In the above, although the embodiments have been described in detail, the scope of the present invention is not limited thereto, and various modifications and improvements by those skilled in the art falling under the scope of the appended claims are also included in the scope of the present invention.

**Claims**

**1.** An ultrasonic diagnostic apparatus (100) comprising:

a display (140);
a memory (1320) configured to store one or more instructions; and

a processor (1310) configured to execute the one or more instructions stored in the memory, wherein the processor (1310) is configured to:

acquire a plurality of pieces of elastic data about an object and then generate a plurality of elastic images based on the plurality of pieces of elastic data, acquire reliability information for elasticity values respectively included in the plurality of elastic images, determine quality information for each of the plurality of elastic images based on a proportion of a region where the reliability information is greater than or equal to a threshold value set by a user input from among the entire region of interest from which the elastic data is acquired, determine at least one recommended elastic image from which an elasticity value is acquired from among the plurality of elastic images based on the quality information, and control the display (140) to display the at least one determined recommended elastic image.

2. The ultrasonic diagnostic apparatus of claim 1, wherein the processor (1310) determines a recommended position from which the elasticity value is acquired based on reliability information for the elasticity values included in the at least one determined recommended elastic image, and controls the display to display the recommended position on the recommended elastic image.

3. The ultrasonic diagnostic apparatus of claim 1, further comprising a user input unit (170) configured to receive a user input selecting any one from among the at least one determined recommended elastic image, wherein the processor (1310) determines at least one recommended position from which the elasticity value is acquired based on reliability information for the elasticity values included in the selected recommended elastic image, and controls the display (140) to display the at least one recommended position on the recommended elastic image.

4. The ultrasonic diagnostic apparatus of claim 3, wherein the processor (1310) controls the display (140) to display a priority of the at least one recommended position when the at least one recommended position is a plurality of recommended positions.

5. The ultrasonic diagnostic apparatus of claim 4, wherein the priority of the recommended positions is determined based on the quality information corresponding to the elastic image including the recommended position and the reliability information corresponding to the recommended position.

6. The ultrasonic diagnostic apparatus of claim 3, wherein the processor (1310) acquires the elasticity value corresponding to the at least one recommended position, and controls the display (140) to display the acquired elasticity value.

7. The ultrasonic diagnostic apparatus of claim 1, wherein the processor (1310) controls the display (140) to display the quality information corresponding to the at least one recommended elastic image.

8. The ultrasonic diagnostic apparatus of claim 1, wherein the processor (1310) determines a representative elasticity value corresponding to the at least one recommended elastic image, and controls the display (140) to display the representative elasticity value.

9. An operating method of an ultrasonic diagnostic apparatus (100), the method comprising operations of:

acquiring a plurality of pieces of elastic data about an object; generating a plurality of elastic images based on the plurality of pieces of elastic data; acquiring reliability information for elasticity values respectively included in the plurality of elastic images; determining quality information for each of the plurality of elastic images based on a proportion of a region where the reliability information is greater than or equal to a threshold value set by a user input from among an entire region of interest from which the elastic data is acquired; determining at least one recommended elastic image from which an elasticity value is acquired from among the plurality of elastic images based on the quality information; and displaying the at least one determined recommended elastic image.

10. The method of claim 9, further comprising operations of:

determining a recommended position from which the elasticity value is acquired based on the reliability information for the elasticity values included in the at least one determined recommended elastic image; and displaying the recommended position on the recommended elastic image.

11. One or more computer-readable recording media in which a program for performing the method of claim 9 is stored.

**Patentansprüche**

1. Ultraschalldiagnosevorrichtung (100), die Folgendes aufweist:

   eine Anzeige (140);
   einen Speicher (1320), der zum Speichern einer oder mehrerer Anweisungen ausgebildet ist; und
   einen Prozessor (1310), der dafür vorgesehen ist, den einen oder die mehreren in dem Speicher gespeicherten Befehle auszuführen,
   wobei der Prozessor (1310) für Folgendes vorgesehen ist:

   eine Vielzahl von elastischen Daten über ein Objekt zu erfassen und dann eine Vielzahl von elastischen Bildern basierend auf der Vielzahl von elastischen Daten zu erzeugen,
   Zuverlässigkeitsinformationen für Elastizitätswerte zu erfassen, die jeweils in der Vielzahl von elastischen Bildern enthalten sind,
   Qualitätsinformationen für jedes der Vielzahl von elastischen Bildern auf der Grundlage eines Anteils eines Bereichs zu bestimmen, in dem die Zuverlässigkeitsinformationen größer oder gleich einem durch eine Benutzereingabe aus dem gesamten interessierenden Bereich, von dem die elastischen Daten erfasst werden, eingestellten Schwellenwert sind,
   wenigstens ein empfohlenes elastisches Bild zu bestimmen, von dem ein Elastizitätswert aus der Vielzahl der elastischen Bilder basierend auf der Qualitätsinformation erfasst wird, und
   die Anzeige (140) so zu steuern, dass sie das wenigstens eine empfohlene elastische Bild anzeigt.

2. Ultraschalldiagnosevorrichtung nach Anspruch 1, wobei der Prozessor (1310) eine empfohlene Position bestimmt, von der der Elastizitätswert auf der Grundlage von Zuverlässigkeitsinformationen für die in dem wenigstens einen bestimmten empfohlenen elastischen Bild enthaltenen Elastizitätswerte erfasst wird, und die Anzeige so steuert, dass sie die empfohlene Position auf dem empfohlenen elastischen Bild anzeigt.

3. Ultraschalldiagnosevorrichtung nach Anspruch 1, die des Weiteren eine Benutzereingabeeinheit (170) aufweist, die so ausgebildet ist, dass sie eine Benutzereingabe empfängt, die ein beliebiges Bild aus dem wenigstens einen bestimmten empfohlenen elastischen Bild auswählt,
   wobei der Prozessor (1310) wenigstens eine empfohlene Position bestimmt, von der aus der Elastizitätswert auf der Grundlage von Zuverlässigkeitsinformationen für die in dem ausgewählten empfohlenen elastischen Bild enthaltenen Elastizitätswerte erfasst wird, und die Anzeige (140) so steuert, dass sie die wenigstens eine empfohlene Position auf dem empfohlenen elastischen Bild anzeigt.

4. Ultraschalldiagnosevorrichtung nach Anspruch 3, wobei der Prozessor (1310) die Anzeige (140) so steuert, dass eine Priorität der wenigstens einen empfohlenen Position angezeigt wird, wenn die wenigstens eine empfohlene Position eine Vielzahl von empfohlenen Positionen ist.

5. Ultraschalldiagnosevorrichtung nach Anspruch 4, wobei die Priorität der empfohlenen Positionen auf der Grundlage der Qualitätsinformation, die dem elastischen Bild entspricht, einschließlich der empfohlenen Position und der Zuverlässigkeitsinformation, die der empfohlenen Position entspricht, bestimmt wird.

6. Ultraschalldiagnosevorrichtung nach Anspruch 3, wobei der Prozessor (1310) den Elastizitätswert entsprechend der wenigstens einen empfohlenen Position erfasst und die Anzeige (140) so steuert, dass sie den erfassten Elastizitätswert anzeigt.

7. Ultraschalldiagnosevorrichtung nach Anspruch 1, wobei der Prozessor (1310) die Anzeige (140) so steuert, dass sie die Qualitätsinformationen anzeigt, die dem wenigstens einen empfohlenen elastischen Bild entsprechen.

8. Ultraschalldiagnosevorrichtung nach Anspruch 1, wobei der Prozessor (1310) einen repräsentativen Elastizitätswert bestimmt, der dem wenigstens einen empfohlenen elastischen Bild entspricht, und die Anzeige (140) so steuert, dass sie den repräsentativen Elastizitätswert anzeigt.

9. Verfahren zum Betreiben einer Ultraschalldiagnosevorrichtung (100), wobei das Verfahren die folgenden Vorgänge aufweist:

Erfassen einer Vielzahl von elastischen Daten über ein Objekt;
Erzeugen einer Vielzahl von elastischen Bildern basierend auf der Vielzahl von elastischen Daten;
Erfassen von Zuverlässigkeitsinformationen für Elastizitätswerte, die jeweils in der Vielzahl der elastischen Bilder enthalten sind;
Bestimmen von Qualitätsinformationen für jedes der Vielzahl von elastischen Bildern auf der Grundlage eines Anteils eines Bereichs, in dem die Zuverlässigkeitsinformationen größer oder gleich einem durch eine Benutzereingabe aus einem gesamten interessierenden Bereich, von dem die elastischen Daten erfasst werden, eingestellten Schwellenwert sind;
Bestimmen wenigstens eines empfohlenen elastischen Bildes, von dem ein Elastizitätswert aus der Vielzahl der elastischen Bilder basierend auf der Grundlage der Qualitätsinformationen erfasst wird; und
Anzeigen des wenigstens einen bestimmten empfohlenen elastischen Bildes.

10. Verfahren nach Anspruch 9, das des Weiteren die folgenden Vorgänge aufweist:

Bestimmen einer empfohlenen Position, von der der Elastizitätswert auf der Grundlage der Zuverlässigkeitsinformationen für die Elastizitätswerte, die in dem wenigstens einen bestimmten empfohlenen elastischen Bild enthalten sind, erfasst wird; und
Anzeigen der empfohlenen Position auf dem empfohlenen elastischen Bild.

11. Ein oder mehrere computerlesbare Aufzeichnungsmedien, auf denen ein Programm zur Durchführung des Verfahrens nach Anspruch 9 gespeichert ist.

**Revendications**

1. Appareil de diagnostic ultrasonore (100), comprenant :

un affichage (140) ;
une mémoire (1320) configurée pour stocker une ou plusieurs instructions ;
un processeur (1310) configuré pour exécuter les une ou plusieurs instructions stockées dans la mémoire,
dans lequel le processeur (1310) est configuré pour :

acquérir une pluralité d'éléments de données élastiques concernant un objet, puis générer une pluralité d'images élastiques sur la base de la pluralité d'éléments de données élastiques,
acquérir des informations de fiabilité pour des valeurs d'élasticité incluses respectivement dans la pluralité d'images élastiques,
déterminer des informations de qualité pour chacune de la pluralité d'images élastiques sur la base d'une proportion d'une région où les informations de fiabilité sont supérieures ou égales à une valeur de seuil définie par une entrée d'utilisateur parmi toute la région d'intérêt à partir de laquelle les données élastiques sont acquises,
déterminer au moins une image élastique recommandée à partir de laquelle une valeur d'élasticité est acquise parmi la pluralité d'images élastiques sur la base des informations de qualité, et
commander l'affichage (140) pour afficher la au moins une image élastique recommandée déterminée.

2. Appareil de diagnostic ultrasonore selon la revendication 1, dans lequel le processeur (1310) détermine une position recommandée à partir de laquelle la valeur d'élasticité est acquise sur la base d'informations de fiabilité pour les valeurs d'élasticité incluses dans la au moins une image élastique recommandée déterminée, et commande l'affichage pour afficher la position recommandée sur l'image élastique recommandée.

3. Appareil de diagnostic ultrasonore selon la revendication 1, comprenant en outre une unité d'entrée d'utilisateur (170) configurée pour recevoir une entrée d'utilisateur sélectionnant une quelconque parmi la au moins une image élastique recommandée déterminée,

dans lequel le processeur (1310) détermine au moins une position recommandée à partir de laquelle la valeur d'élasticité est acquise sur la base d'informations de fiabilité pour les valeurs d'élasticité incluses dans l'image élastique recommandée sélectionnée, et commande l'affichage (140) pour afficher la au moins une position recommandée sur l'image élastique recommandée.

4. Appareil de diagnostic ultrasonore selon la revendication 3, dans lequel le processeur (1310) commande l'affichage (140) pour afficher une priorité de la au moins une position recommandée lorsque la au moins une position recommandée est une pluralité de positions recommandées.

5. Appareil de diagnostic ultrasonore selon la revendication 4, dans lequel la priorité des positions recommandées est déterminée sur la base des informations de qualité correspondant à l'image élastique incluant la position recommandée et des informations de fiabilité correspondant à la position recommandée.

6. Appareil de diagnostic ultrasonore selon la revendication 3, dans lequel le processeur (1310) acquiert la valeur d'élasticité correspondant à la au moins une position recommandée, et commande l'affichage (140) pour afficher la valeur d'élasticité acquise.

7. Appareil de diagnostic ultrasonore selon la revendication 1, dans lequel le processeur (1310) commande l'affichage (140) pour afficher les informations de qualité correspondant à la au moins une image élastique recommandée.

8. Appareil de diagnostic ultrasonore selon la revendication 1, dans lequel le processeur (1310) détermine une valeur d'élasticité représentative correspondant à la au moins une image élastique recommandée, et commande l'affichage (140) pour afficher la valeur d'élasticité représentative.

9. Procédé de fonctionnement d'un appareil de diagnostic ultrasonore (100), le procédé comprenant les opérations suivantes consistant à :

> acquérir une pluralité d'éléments de données élastiques concernant un objet ;
> générer une pluralité d'images élastiques sur la base de la pluralité d'éléments de données élastiques ;
> acquérir des informations de fiabilité pour des valeurs d'élasticité incluses respectivement dans la pluralité d'images élastiques ;
> déterminer des informations de qualité pour chacune de la pluralité d'images élastiques sur la base d'une proportion d'une région où les informations de fiabilité sont supérieures ou égales à une valeur de seuil définie par une entrée d'utilisateur parmi toute une région d'intérêt à partir de laquelle les données élastiques sont acquises ;
> déterminer au moins une image élastique recommandée à partir de laquelle une valeur d'élasticité est acquise parmi la pluralité d'images élastiques sur la base des informations de qualité ; et
> afficher la au moins une image élastique recommandée déterminée.

10. Procédé selon la revendication 9, comprenant en outre les opérations consistant à :

> déterminer une position recommandée à partir de laquelle la valeur d'élasticité est acquise sur la base des informations de fiabilité pour les valeurs d'élasticité incluses dans la au moins une image élastique recommandée déterminée ; et
> afficher la position recommandée sur l'image élastique recommandée.

11. Un ou plusieurs supports d'enregistrement lisibles par ordinateur dans lesquels un programme pour la mise en oeuvre du procédé selon la revendication 9 est stocké.

**FIG. 1**

EP 4 082 441 B1

FIG. 2

100c

145

40

20

(c)

100b

121

122

165

172

171

(b)

100a

121

122

(a)

**FIG. 3**

```
              ( START )
                   |
                   v
  +---------------------------------+
  |      GENERATE  PLURALITY  OF    |
  |    ELASTIC  IMAGES  FOR  OBJECT |~S310
  +---------------------------------+
                   |
                   v
  +---------------------------------+
  |  ACQUIRE  QUALITY  INFORMATION  FOR |
  |    PLURALITY  OF  ELASTIC  IMAGES   |~S320
  +---------------------------------+
                   |
                   v
  +---------------------------------+
  |   DETERMINE  RECOMMENDED  ELASTIC   |
  | IMAGE  BASED  ON  QUALITY  INFORMATION |~S330
  +---------------------------------+
                   |
                   v
  +---------------------------------+
  |      DISPLAY  RECOMMENDED        |
  |         ELASTIC  IMAGE           |~S340
  +---------------------------------+
                   |
                   v
               ( END )
```

## FIG. 4

Quality index

411 ⟶     421    ⟹ 0.11

412 ⟶     ⟹ 0.39

413 ⟶     ⟹ 0.53

414 ⟶     ⟹ 0.87

419 ⟶     ⟹ 0.41

**FIG. 5**

**FIG. 6**

START

↓

DETERMINE RECOMMENDED POSITION FROM WHICH ELASTICITY VALUE IS ACQUIRED FROM RECOMMENDED ELASTIC IMAGE ~S610

↓

DISPLAY RECOMMENDED POSITION AND ELASTICITY VALUE CORRESPONDING TO RECOMMENDED POSITION ~S620

↓

END

## FIG. 7

**FIG. 8**

EP 4 082 441 B1

FIG. 9

**FIG. 10**

**FIG. 11**

FIG. 12

EP 4 082 441 B1

FIG. 13

**EP 4 082 441 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016331345 A1
- KR 20170085516 A